Europäisches Patentamt

European Patent Office

Office européen des brevets

(19)

(11) **EP 0 640 285 B1**

(12) **EUROPÄISCHE PATENTSCHRIFT**

(45) Veröffentlichungstag und Bekanntmachung des Hinweises auf die Patenterteilung:
08.07.1998 Patentblatt 1998/28

(51) Int. Cl.$^6$: **A01N 37/36**, A01N 31/00, C11D 3/48

(21) Anmeldenummer: 94250197.4

(22) Anmeldetag: 10.08.1994

(54) **Waschende Händedesinfektions- und Händedekontaminationsmittel auf Basis naturidentischer, aromatischer Alkohole**

Hygienic handwash for disinfecting and decontaminating hands based on natural aromatic alcohols

Agent de lavage pour la désinfection et décontamination des mains à base d'alcools aromatiques naturels

(84) Benannte Vertragsstaaten:
**DE ES FR GB IT**

(30) Priorität: **30.08.1993 DE 4329517**

(43) Veröffentlichungstag der Anmeldung:
**01.03.1995 Patentblatt 1995/09**

(73) Patentinhaber:
**Schülke & Mayr GmbH**
**22851 Norderstedt (DE)**

(72) Erfinder:
• **Eggensperger, Heinz, Dr.**
**D-22397 Hamburg (DE)**

• **Goroncy-Bermes, Peter, Dr.**
**D-22926 Ahrensburg (DE)**
• **Behrends, Sabine**
**D-25421 Pinneberg (DE)**
• **Puchstein, Burghard**
**D-22309 Hamburg (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 016 319          EP-A- 0 423 922**

**Beschreibung**

Die Bedeutung der Händedesinfektion zum Zwecke der Verhütung nosokomialer Infektionen in Krankenhäusern und medizinischen Praxen ist seit Semmelweis unbestritten.

In der pharmazeutischen Industrie und der Lebensmittelindustrie hat die Händedesinfektion und -dekontamination vor allem die Aufgabe, eine mögliche Kontamination der hergestellten Produkte durch unerwünschte Keime zu verhindern. Für die Behandlung der Hände werden in diesen Bereichen häufig Präparate verwendet, die gleichzeitig reinigen und desinfizieren. Derartige Präparate sind heute unter der Bezeichnung "waschende Händedesinfektionsmittel und Händedekontaminationsmittel" bekannt.

Durch das Abspülen der Hände nach dem Reinigungs- und Desinfektions-bzw. Dekontaminationsvorgang gelangen Produktbestandteile letztlich ins Abwasser. Damit erhält auch die Frage ihres biologischen Abbaus für den Umweltschutz hohe Bedeutung.

Das in der Patentschrift DE 31 17 792 C3 beschriebene wäßrige Händedesinfektionsmittel enthält als wesentlichen Wirkstoff 2-Phenylphenol. Obwohl dieses Phenol an sich gut abbaubar ist, kann jedoch nicht ausgeschlossen werden, daß es in chlorhaltigem Abwasser zu einem schwer abbaubaren Polychlorphenol umgesetzt wird.

Bei der Verwendung als Händedekontaminationsmittel werden Phenole - auch das 2-Phenylphenol - zunehmend abgelehnt, weil nicht mit absoluter Sicherheit ausgeschlossen werden kann, daß in der Praxis Spuren des Phenols auf die hergestellten Pharmazeutika und Lebensmittel übertragen werden.

Ferner sind aus der DE-AS-11 05 549 desinfizierende Wasch- und Reinigungsmittel bekannt, die ein Gemisch von freier Milchsäure mit anderen Desinfektionsmittelwirkstoffen wie Aldehyden, Phenolen oder Quecksilberverbindungen, oberflächenaktiven, säurebeständigen anionischen, nichtionischen, kationischen oder ampholytischen Substanzen enthalten. Diese Mittel sind jedoch ebenfalls arzneimittel- und lebensmittelrechtlich bedenklich bzw. nicht akzeptabel oder aus ökologischer Sicht unerwünscht.

Ein bekanntes und hochwirksames Verfahren zur Händedesinfektion besteht im Einreiben der Hände mit Alkoholen wie Ethanol, Isopropanol oder n-Propanol oder einem Gemisch derselben. Dieses Verfahren ist dann nicht praxisgerecht, wenn die Hände mit Schmutz aller Art sichtbar belastet sind, wie es z.B. in der Pathologie und der Lebensmittelindustrie oft der Fall ist.

Außerhalb des deutschsprachigen Raums wird die waschende Händedesinfektion ohnehin immer noch der Einreibemethode vorgezogen.

Die CEN (Comité Européen de Normalisation) hat bei der Festlegung der zukünftigen Verfahren zur Prüfung von Händedesinfektionsmitteln in Europa diese Vorgehensweise berücksichtigt und daraufhin eine neue Testmethode für die waschenden Händedesinfektionsmittel (Hygienic Handwash) vorgeschlagen.

Daher ist es wünschenswert, daß waschende Händedesinfektionsmittel nicht nur die Bedingungen der Händedekontamination erfüllen, wie sie in der Lebensmittelindustrie gefordert werden, sondern auch diese neuen Anforderungen der CEN-Methode.

Mischungen aus Tensiden und den Alkoholen Ethanol, Isopropanol und n-Propanol erfüllen beide Testbedingungen, wenn der Anteil dieser Alkohole mehr als 45 Gew.-% beträgt. Diese Tensid-Alkohol-Gemische sind jedoch schlecht hautverträglich.

Die Erfindung hat sich deshalb die Aufgabe gestellt, die Bedingungen der CEN-Methode und der Händedekontamination mit einem waschenden Händedesinfektionsmittel zu erreichen, das sich durch eine hohe Hautverträglichkeit auszeichnet und keine phenolischen Wirkstoffe enthält.

Diese Aufgabe wird gemäß Anspruch 1 überraschenderweise durch ein waschendes Händedesinfektions- und Händedekontaminationsmittel gelöst, das eine wäßrige Lösung umfaßt, die

a) 5 bis 35, vorzugsweise 10 bis 30 Gew.-% $C_1$-$C_{19}$-Alkylalkohol,
b) 0,1 bis 10,0, vorzugsweise 0,5 bis 5,0 Gew.-% in der Natur vorkommenden $C_6$-Aryl-$C_1$-$C_8$-alkylalkohol,
c) 0,5 bis 45, vorzugsweise 0,5 bis 15 Gew.-% Sulfosuccinat, Betain oder Fettalkoholethersulfat oder eine Mischung derselben und
d) 0,1 bis 5,0, vorzugsweise 0,1 bis 3,0 Gew.-% hautverträgliche $\alpha$-Hydroxycarbonsäure als Hautschutzkomponente und zur Einstellung auf einen pH-Wert von 2 bis 7 enthält.

Bevorzugten Ausführungsformen sind Gegenstand der Unteransprüche.

Als $C_1$-$C_{19}$-Alkylalkohol, vorzugsweise $C_1$-$C_{12}$- und insbesondere $C_1$-$C_6$-Alkylalkohol sind Verbindungen wie Ethanol, Isopropanol oder n-Propanol oder eine Mischung derselben bevorzugt.

Die $C_6$-Aryl-$C_1$-$C_8$-alkylalkohole sind solche mit unverzweigten Alkylgruppen und vorzugsweise $C_6$-Aryl-$C_1$-$C_6$-alkylalkohole und insbesondere $C_6$-Aryl-$C_1$-$C_3$-alkylalkohole wie Benzylalkohol, Phenylethylalkohol, Phenylpropylalkohol oder eine Mischung derselben.

Erfindungsgemäß bevorzugte $\alpha$-Hydroxycarbonsäuren sind Milchsäure und Mandelsäure.

2

Vorzugsweise handelt es sich bei dem Sulfosuccinat um das Di-Natriumsalz des Sulfobernsteinsäurelaurylesters, wobei eine COOH-Gruppe verestert ist und die andere COOH-Gruppe und die $SO_3H$-Gruppe als Anion (Salzform) vorliegen, mit 1 - 4 EO (Ethylenoxideinheiten), bei dem Betain um Cocamidopropylbetain oder Laurylbetain und bei dem Fettalkoholethersulfat um Natriumlaurylethersulfat, Magnesiumlaurylethersulfat, Monoethanolaminethersulfat oder Ammoniumlaurylethersulfat oder einer Mischung derselben, jeweils mit 1 - 6 EO. Der pH-Wert der Lösung liegt vorzugsweise im Bereich von 3 bis 6.

Überraschend ist, daß diese Kombination ausgewählter Komponenten, die jeweils allein eingesetzt keine ausreichende Wirkung besitzen bzw. allenfalls in wesentlich höheren Einsatzkonzentrationen eine Wirkung zeigen, zu einem Mittel führt, das sowohl die neuen Bedingungen der CEN-Methode als auch die der Händedekontamination erfüllt und sich zudem durch eine ausgezeichnete Hautverträglichkeit und gute biologische Abbaubarkeit auszeichnet.

Vorteilhaft ist auch, daß die alkoholische Wirkstoffbasis ausschließlich aus Alkoholen besteht, die in der Natur vorkommen und deren toxikologische Eigenschaften hinreichend bekannt sind.

Den erfindungsgemäß verwendeten Mischungen können weitere Hilfsstoffe zugesetzt werden, z.B. Hautschutzstoffe wie Allantoin in einer Menge von vorzugsweise 0,1 bis 0,3 Gew.-%, sogenannte Rückfetter wie Polyolfettsäureester oder Laurylalkohol mit 2 EO in einer Menge von vorzugsweise 0,5 bis 3,0 Gew.-%, Verdickungsmittel wie Hydroxyethylcellulose oder deren Derivate in einer Menge von vorzugsweise 0,5 bis 2,0 Gew.-%, Farbstoffe in einer Menge von vorzugsweise 0,001 bis 0,1 Gew.-% oder Parfüms in einer Menge von vorzugsweise 0,05 bis 1,0 Gew.-%. Die Mengenangaben beziehen sich jeweils auf die Gesamtmischung.

Beispielsweise können die folgenden Basis-Formulierungen A bis C als Desinfektionslösung verwendet werden:

**Formulierung A**

| | |
|---|---|
| 15 Gew.-% | 1-Propanol |
| 12 Gew.-% | 2-Propanol |
| 2 Gew.-% | 2-Phenethylalkohol |
| 10 Gew.-% | Di-Natriumsalz des Sulfobernsteinsäurelaurylesters mit 1 - 4 EO |
| 3 Gew.-% | Cocamidopropylbetain |
| 0,5 Gew.-% | Milchsäure |
| Rest | Wasser |

**Formulierung B**

| | |
|---|---|
| 10 Gew.-% | 1-Propanol |
| 8 Gew.-% | 2-Propanol |
| 2 Gew.-% | 3-Phenylpropanol-1 |
| 1 Gew.-% | Di-Natriumsalz des Sulfobernsteinsäurelaurylesters mit 1 - 4 EO |
| 9 Gew.-% | Natriumlaurylethersulfat mit 1 - 6 EO |
| 0,5 Gew.-% | Milchsäure |
| Rest | Wasser |

**Formulierung C**

| | |
|---|---|
| 10 Gew.-% | 1-Propanol |
| 8 Gew.-% | 2-Propanol |
| 2 Gew.-% | 3-Phenylpropanol-1 |
| 1 Gew.-% | Di-Natriumsalz des Sulfobernsteinsäurelaurylesters mit 1 - 4 EO |
| 9 Gew.-% | Natriumlaurylethersulfat mit 1 - 6 EO |
| 1 Gew.-% | Mandelsäure |
| Rest | Wasser |

Die erfindungsgemäßen Händedesinfektions- und Händedekontaminationsmittel werden hergestellt, indem der Arylalkylalkohol bei Raumtemperatur in dem Alkylalkohol gelöst wird. Anschließend werden die übrigen Komponenten zugefügt, wobei die Säure zum Schluß zugesetzt wird.

Die Wirksamkeit dieser Mittel wurde sowohl nach der neuen Methode der CEN als auch der bekannten Methode der Händedekontamination geprüft.

**Hygienisches Händewaschen nach CEN**

**1. Anforderungen**

Die mittlere Reduktion der Mikroorganismen durch Behandlung der Hände mit einem Produkt für die hygienische Händewaschung muß signifikant größer sein als die Keimreduktion, die mit der Referenzlösung (Sapo Kalinus, Eur. Pharm, 20 % Gew./Vol.) erzielt wird. Die Anwendungszeit darf 60 Sekunden nicht überschreiten.

Die Reduktionsfaktoren ermitteln sich aus den Differenzen der Vor- und Nachwerte, üblicherweise durch den dekadischen Logarithmus ausgedrückt:

$$\log RF = \log \text{Vorwert} - \log \text{Nachwert}$$

**2. Probanden**

Für diesen Versuch werden 15 Probanden mit gesunder Haut und kurzen Fingernägeln benötigt.

**3. Testorganismus**

Der Standard-Testorganismus ist Escherichia coli ATCC 11229. E. coli wird in zwei Röhrchen mit je 5 ml CS-Lösung (CSL; Caseinpepton-Sojabohnenmehlpepton-Lösung; Nährmedium zur Anzucht von Bakterien) 18 bis 24 Stunden lang bei 36°C angezüchtet. Der Inhalt dieser Röhrchen wird dann in je 1 l CS-Lösung gegeben und wiederum 18 bis 24 Stunden lang bei 36°C $\pm$ 1°C bebrütet. Danach werden die beiden Suspensionen gemischt (= 2 l Gesamtvolumen).

Die Keimzahl soll $2 \times 10^8$ bis $2 \times 10^9$ KBE/ml (koloniebildende Einheiten pro ml) betragen.

**4. Kontamination der Hände**

Die Hände werden 1 Minute lang mit der Referenzlösung (Sapo Kalinus, 20 % Gew./Vol.) gewaschen und danach sofort mit Papierhandtüchern getrocknet. Anschließend werden die Hände 5 Sekunden lang in die Keimsuspension getaucht und an der Luft getrocknet (3 Minuten). Nach Trocknung der Hände werden die Vorwerte bestimmt.

**5. Händswaschen mit der Referenzlösung (R)**

5 ml Sapo Kalinus werden in die angefeuchteten Hände gegeben, die nach einem bestimmten Procedere gewaschen werden müssen. Hierbei werden die folgenden Waschschritte vollzogen, wobei jeder dieser Schritte 5 Hin-und-Her-Bewegungen umfaßt und gegebenenfalls die Hände gewechselt werden. Zuerst werden die Handflächen aneinander gerieben. Dann werden die Handflächen der einen Hand an dem Handrücken der anderen Hand gerieben. Als nächstes werden wiederum die Handflächen aneinander gerieben, wobei die Finger beider Hände diesmal ineinander verschränkt sind. Anschließend werden die Fingerrücken der einen Hand an der Handfläche der anderen Hand gerieben, wobei die Finger beider Hände hakenförmig ineinander eingreifen. Ferner wird der Daumen der einen Hand von der anderen Hand ergriffen und mittels Drehbewegungen an der Handfläche der die Faust bildenden Hand gerieben. Schließlich werden die eingeklappten Fingern der einen Hand in der Handfläche der anderen Hand drehend hin-und-her bewegt. Nach genau 60 Sekunden werden die Hände abgespült. Anschließend erfolgt die Bestimmung der Nachwerte.

**6. Händewaschen mit der Prüflösung (P)**

Dieser Vorgang erfolgt entsprechend der obigen Vorschrift zur Referenzlösung. Der Mittelwert der log Reduktionsfaktoren des Prüfpräparates muß signifikant besser sein als der Mittelwert der log Reduktionsfaktoren der Referenzlösung Sapo kalinus.

**7. Prüfung der Signifikanz**

Zur Überprüfung der Signifikanz wird der Wilcoxon-Test für den paarigen Vergleich verbundener Stichproben (Signifikanz-Niveau: P = 0,01) bei einseitiger Fragestellung angewandt.

Tabelle 1

| Statistischer, paarweiser Vergleich der bei R und P erhobenen log-Rf-Werte (Wilcoxon-Test) | | | | | |
|---|---|---|---|---|---|
| Proband | P | R | P-R | Rang der Differenzen | Rang mit Vorzeichen |
| 1 | 3,98 | 3,33 | 0,65 | 11 | 11 |
| 2 | 3,21 | 2,68 | 0,53 | 8 | 8 |
| 3 | 4,38 | 2,90 | 1,48 | 15 | 15 |
| 4 | 3,34 | 2,77 | 0,57 | 9 | 9 |
| 5 | 3,55 | 2,70 | 0,85 | 14 | 14 |
| 6 | 3,60 | 2,83 | 0,77 | 13 | 13 |
| 7 | 2,79 | 2,52 | 0,27 | 6 | 6 |
| 8 | 2,72 | 2,73 | -0,01 | 1 | - 1 |
| 9 | 2,69 | 2,80 | -0,11 | 3 | - 3 |
| 10 | 3,26 | 2,57 | 0,69 | 12 | 12 |
| 11 | 3,11 | 2,87 | 0,24 | 4 | 4 |
| 12 | 2,61 | 3,03 | -0,42 | 7 | - 7 |
| 13 | 2,49 | 2,23 | 0,26 | 5 | 5 |
| 14 | 3,21 | 2,63 | 0,58 | 10 | 10 |
| 15 | 3,25 | 3,30 | -0,05 | 2 | - 2 |
| Rangsumme (+) = 107<br>Rangsumme (-) = 13<br>T = 13 (kleinere Rangsumme) | | | | | |

Die kleinere Rangsumme (T = 13) wird mit dem in Tabelle 2 angegebenen Wert bei 15 Paaren und einem Signifikanzniveau 0,01 verglichen (hier 19). Wenn T kleiner als der Tabellenwert ist, ist P signifikant besser als R.

Tabelle 2

| Vorzeichentest für Paardifferenzen nach Wilcoxon Signifikanzschranken für die kleinere Summe der Ränge mit gleichen Vorzeichen (T) bei einseitiger Fragestellung | | | |
|---|---|---|---|
| n (Zahl der Paare mit einem Unterschied $\neq 0$) | Signifikanzniveau | | |
| | 0,05 | 0,01 | 0,001 |
| 12 | 17 | 9 | 2 |
| 13 | 21 | 12 | 4 |
| 14 | 25 | 15 | 6 |
| 15 | 30 | 19 | 8 |

**Anleitung für den Vorzeichen-Rangtest:**

1. Es wird die Differenz jedes Wertepaares bestimmt.
2. Ohne Beachtung des Vorzeichens werden den Differenzen nach ihrer absoluten Größe Rangplätze zugeteilt.
3. Zu jeder Rangzahl wird das Vorzeichen des entsprechenden Differenzwertes hinzugefügt.

4. Es wird die Summe der (+)-Rangzahlen und der (-)-Rangzahlen gebildet.

5. Es wird die Anzahl der Paare bestimmt, deren Differenz nicht 0 ergibt (= n).

6. In Tabelle 2 wird der Tabellenwert in Zeile n und Spalte 0,01 aufgesucht. Wenn die kleinere Rangsumme T gleichgroß wie oder kleiner als der Tabellenwert ist, so ist der mittlere log Rf von P signifikant größer als der von R.

## Händedekontamination

Versuche unter praxisnahen Bedingungen (Zbl. Bakt. Hyg. B 182, 562 - 570; 1986).

## Prinzip

Als Maß für die Wirkung im praxisnahen Versuch dient die Verringerung der Keimabgabe von den Fingerkuppen der künstlich kontaminierten Hand. Die Größe dieser Reduktion ergibt sich aus dem Verhältnis der Keimmengen, die vor und nach der Händedekontamination von den Fingerkuppen in eine Sammelflüssigkeit abgegeben werden.

## Probanden (= PR)

Die Versuche werden mit 20 verschiedenen PR durchgeführt. Für die Auswertung müssen die Werte von mindestens 18 PR zur Verfügung stehen; sofern die Standardabweichung mehr als 50 % des Mittelwertes der Reduktionsraten ausmacht, ist die Zahl der PR in einem weiteren Test zu erhöhen. Personen mit starken Hyperkeratosen, Hautverletzungen sowie mit langen Fingernägeln sind nicht geeignet.

## Testkeim

Escherichia coli ATCC 11229

## Kontamination

Nach dem Waschen der Hände mit einer nicht antimikrobiell wirkenden Seife unter fließendem, handwarmem Wasser für 2 Minuten werden die Hände mit Einmalhandtüchern getrocknet. Danach werden sie unter Einbeziehung des Daumens 5 Sekunden lang bis zur Mitte der Mittelhand in eine Suspension von E. coli getaucht, die durch Inkubation des Testkeimes 24 Stunden lang bei 37°C 2 l CS-Lösung hergestellt worden ist. Die Suspension muß mindestens $10^8$ KBE/ml enthalten, was durch eine quantitative Oberflächenstruktur auf CSA nachzuweisen ist. Nach kurzem Abtropfen werden die Hände zum Lufttrocknen 3 Minuten lang horizontal gehalten, wobei durch Spreizen der Finger und langsames Hin- und Herdrehen eine Tropfenbildung vermieden werden soll.

## Dekontaminationsverfahren

Bestimmung der abgegebenen KBE vor der Händedekontamination = Vorwerte (VW):
Für die Bestimmung der Anzahl KBE, welche die Fingerkuppen der kontaminierten Hände vor der Dekontamination abgeben, werden die Kuppen der Finger 1 - 5 jeder Hand in eine mit 10 ml Sammelflüssigkeit (CSL + Inaktivierungssubstanzen) gefüllte Kunststoff-Petrischale (Durchmesser ca. 9 cm) getaucht und auf dem Boden der Schale unter kreisenden und reibenden Bewegungen 1 Minute lang ausgeknetet. Aus Verdünnungen 1:1000 und 1:10000 in CSL - Inaktivierungssubstanzen) dieser Sammelflüssigkeit werden je 0,1 ml auf CSA (mit Zusatz von 0,05 % Na-Desoxycholat) ausgespatelt.
Anschließend wird die Händedekontamination vorgenommen. Die Einwirkung des Dekontaminationsmittels soll 30 Sekunden betragen. Danach werden die Hände exakt 15 Sekunden lang sorgfältig unter fließendem, handwarmem Leitungswasser abgespült.
Bestimmung der abgegebenen KBE nach der Händekontamination = Nachwerte (NW):
Um festzustellen, wie viele KBE des Testkeimes die Fingerkuppen der kontaminierten Hände nach der Anwendung des Dekontaminationsverfahrens noch abgeben, wird analog der Vorwert-Erhebung vorgegangen. Da aber nunmehr niedrige Werte zu erwarten sind, werden die Portionen von 0,1 ml der unverdünnten Sammelflüssigkeit sowie 0,1 ml einer Verdünnung von 1 : 10 (in CSL + Inaktivierungssubstanzen) auf CSA-Platten (mit Zusatz von 0,05 % Na-Desoxycholat) ausgespatelt.
Bei der praktischen Anwendung wird das Präparat nach der 30 Sekunden-Dekontamination mit etwas Wasser aufgeschäumt und zur Händereinigung eingesetzt.

**Inkubation**

Alle Zählplatten werden 48 Stunden lang bei 37°C aerob bebrütet und danach ausgezählt.

**Bewertung der Ergebnisse des Versuches unter praxisnahen Bedingungen**

Prinzip:
Ein Verfahren für die Händedekontamination wird als geeignet angesehen, wenn es im Durchschnitt die Keimabgabe um mindestens 3,5 Zehnerpotenzen reduziert.

<u>**Ergebnisse**</u>

Die Ergebnisse des Versuchs unter praxisnahen Bedingungen (CEN) sind in Tabelle 1 zusammengefaßt. Nach 60 Sekunden Einwirkzeit konnte eine Keimreduktion (3,21 log-Stufen) erreicht werden. Dies war signifikant besser als das Ergebnis der Referenzlösung. Im praxisnahen Versuch gemäß der DGHM-Richtlinie zur Händedekontamination wurden die Anforderungen ebenfalls erfüllt (Tabelle 2). Nach einer Einreibezeit von 30 Sekunden wurde eine Keimreduktion in Höhe von 3,64 log-Stufen erzielt.

**Tabelle 3:** Bestimmung der mikrobiziden Wirksamkeit unter praxisnahen Bedingungen gemäß der bei CEN vorgeschlagenen Methode

| Proband Nr. | Referenzlösung (R) | | | Prüflösung (P) | | |
|---|---|---|---|---|---|---|
| | VW | – NW | = RF | VW | – NW | = RF |
| 1 | 6,58 | 3,25 | 3,33 | 6,75 | 2,77 | 3,98 |
| 2 | 6,30 | 3,62 | 2,68 | 6,30 | 3,09 | 3,21 |
| 3 | 5,29 | 2,39 | 2,90 | 6,52 | 2,14 | 4,38 |
| 4 | 5,51 | 2,74 | 2,77 | 5,97 | 2,63 | 3,34 |
| 5 | 5,88 | 3,18 | 2,70 | 5,99 | 2,44 | 3,55 |
| 6 | 5,82 | 2,99 | 2,83 | 6,05 | 2,45 | 3,60 |
| 7 | 6,06 | 3,54 | 2,52 | 5,97 | 3,18 | 2,79 |
| 8 | 5,91 | 3,18 | 2,73 | 5,64 | 2,92 | 2,72 |
| 9 | 5,85 | 3,05 | 2,80 | 5,71 | 3,02 | 2,69 |
| 10 | 6,21 | 3,64 | 2,57 | 6,38 | 3,12 | 3,26 |
| 11 | 6,09 | 3,22 | 2,87 | 5,81 | 2,70 | 3,11 |
| 12 | 6,22 | 3,19 | 3,03 | 5,93 | 3,32 | 2,61 |
| 13 | 5,51 | 3,28 | 2,23 | 5,48 | 2,99 | 2,49 |
| 14 | 5,86 | 3,23 | 2,63 | 6,26 | 3,05 | 3,21 |
| 15 | 5,85 | 2,55 | 3,30 | 6,02 | 2,77 | 3,25 |
| Ø | 5,92 – | 3,13 = | 2,79 | 6,05 – | 2,84 = | 3,21 |

Einwirkzeit     = 60 Sek;     Menge: 5 ml;     die Hände wurden     vorher angefeuchtet.

P ist signifikant besser als R.

Signifikanzniveau  = 0,01

Rangsumme (-)    = 13 < T = 19  (siehe Tabelle 1)

**Tabelle 4:** **Bestimmung der mikrobiziden Wirksamkeit unter praxisnahen Bedingungen gemäß der DGHM-Richtlinie zur Händedekontamination**

| Proband Nr. | Prüflösung (P) | | |
|---|---|---|---|
| | VW − | NW = | RF |
| 1 | 6,35 | 3,14 | 3,21 |
| 2 | 5,83 | 2,47 | 3,36 |
| 3 | 5,91 | 2,18 | 3,73 |
| 4 | 5,56 | 2,91 | 2,65 |
| 5 | 6,12 | 3,05 | 3,07 |
| 6 | 6,17 | 2,28 | 3,89 |
| 7 | 6,28 | 2,55 | 3,73 |
| 8 | 5,53 | 1,75 | 3,78 |
| 9 | 6,38 | 2,36 | 4,02 |
| 10 | 5,65 | 1,64 | 4,01 |
| 11 | 6,05 | 2,07 | 3,98 |
| 12 | 6,29 | 2,24 | 4,05 |
| 13 | 6,04 | 2,42 | 3,62 |
| 14 | 5,72 | 1,69 | 4,03 |
| 15 | 5,68 | 2,50 | 3,18 |
| 16 | 6,17 | 2,17 | 4,00 |
| 17 | 6,07 | 1,63 | 4,44 |
| 18 | 5,38 | 1,75 | 3,63 |
| 19 | 5,73 | 2,81 | 2,92 |
| 20 | 5,59 | 2,19 | 3,40 |
| Ø | 5,93 − | 2,29 = | 3,64 |

**Patentansprüche**

1. Waschendes Händedesinfektions- und Händedekontaminationsmittel, dadurch gekennzeichnet, daß es eine wäßrige Lösung umfaßt, die

   a) 5 bis 35 Gew.-% $C_1$-$C_{19}$-Alkylalkohol,
   b) 0,1 bis 10,0 Gew.-% in der Natur vorkommenden $C_6$-Aryl-$C_1$-$C_8$-alkylalkohol,
   c) 0,5 bis 45 Gew.-% Sulfosuccinat, Betain oder Fettalkoholethersulfat oder eine Mischung derselben und
   d) 0,1 bis 5,0 Gew.-% hautverträgliche $\alpha$-Hydroxycarbonsäure als Hautschutzkomponente und zur Einstellung auf einen pH-Wert von 2 bis 7 enthält.

2.  Waschendes Händedesinfektions- und Händedekontaminationsmittel nach Anspruch 1, dadurch gekennzeichnet, daß es

    a) 10 bis 30 Gew.-% $C_1$-$C_{19}$-Alkylalkohol,
    b) 0,5 bis 5,0 Gew.-% in der Natur vorkommenden $C_6$-Aryl-$C_1$-$C_8$-alkylalkohol,
    c) 0,5 bis 15 Gew.-% Sulfosuccinat, Betain oder Fettalkoholethersulfat oder eine Mischung derselben und
    d) 0,1 bis 3,0 Gew.-% hautverträgliche $\alpha$-Hydroxycarbonsäure enthält.

3.  Waschendes Händedesinfektions- und Händedekontaminationsmittel nach Anspruch 1 oder 2, dadurch gekennzeichnet, daß es als

    a) $C_1$-$C_{19}$-Alkylalkohol Ethanol, Isopropanol oder n-Propanol oder eine Mischung derselben,
    b) in der Natur vorkommenden $C_6$-Aryl-$C_1$-$C_8$-alkylalkohol Benzylalkohol, Phenylethylalkohole, Phenylpropyl-alkohole oder eine Mischung derselben,
    c) Sulfosuccinat das Di-Natriumsalz des Sulfobernsteinsäurelaurylesters mit 1 bis 4 EO,
    Betain Cocamidopropylbetain, Laurylbetain oder eine Mischung derselben,
    Fettalkoholethersulfat Natriumlaurylethersulfat, Magnesiumlaurylethersulfat Monoethanolaminethersulfat oder Ammoniumlaurylethersulfat oder einer Mischung derselben, jeweils mit 1 bis 6 EO und als
    d) $\alpha$-Hydroxycarbonsäure Milchsäure oder Mandelsäure enthält.

4.  Waschendes Händedesinfektions- und Händedekontaminationsmittel nach einem der Ansprüche 1 bis 3, dadurch gekennzeichnet, daß es

    | | |
    |---|---|
    | 15 Gew.-% | 1-Propanol, |
    | 12 Gew.-% | 2-Propanol, |
    | 2 Gew.-% | 2-Phenethylalkohol, |
    | 10 Gew.-% | Di-Natriumsalz des Sulfobernsteinsäurelaurylesters mit 1 bis 4 EO, |
    | 3 Gew.-% | Cocamidopropylbetain und |
    | 0,5 Gew.-% | Milchsäure |

    enthält.

5.  Waschendes Händedesinfektions- und Händedekontaminationsmittel nach einem der Ansprüche 1 bis 3, dadurch gekennzeichnet, daß es

    | | |
    |---|---|
    | 10 Gew.-% | 1-Propanol |
    | 8 Gew.-% | 2-Propanol |
    | 2 Gew.-% | 3-Phenylpropanol-1 |
    | 1 Gew.-% | Di-Natriumsalz des Sulfobernsteinsäurelaurylesters mit 1 bis 4 EO, |
    | 9 Gew.-% | Natriumlaurylethersulfat mit 1 - 6 EO |
    | 0,5 Gew.-% | Milchsäure |

    enthält.

6.  Waschendes Händedesinfektions- und Händedekontaminationsmittel nach einem der Ansprüche 1 bis 3, dadurch gekennzeichnet, daß es

    | | |
    |---|---|
    | 10 Gew.-% | 1-Propanol |
    | 8 Gew.-% | 2-Propanol |
    | 2 Gew.-% | 3-Phenylpropanol-1 |
    | 1 Gew.-% | Di-Natriumsalz des Sulfobernsteinsäurelaurylesters mit 1 bis 4 EO, |
    | 9 Gew.-% | Natriumlaurylethersulfat mit 1 - 6 EO |
    | 1 Gew.-% | Mandelsäure |

    enthält.

7.  Waschendes Händedesinfektions- und Händedekontaminationsmittel nach einem der Ansprüche 1 bis 5, dadurch gekennzeichnet, daß der pH-Wert im Bereich von 3 bis 6 liegt.

8. Verfahren zur Herstellung eines waschenden Händedesinfektions-und Händedekontaminationsmittels gemäß einem der Ansprüche 1 bis 7, dadurch gekennzeichnet, daß der Arylalkylalkohol bei Raumtemperatur in dem Alkylalkohol gelöst wird und anschließend die übrigen Komponenten zugefügt werden, wobei die Säure zum Schluß zugesetzt wird.

9. Verwendung einer Lösung gemäß einem der Ansprüche 1 bis 7 als waschendes Händedesinfektions- und Händedekontaminationsmittel.

**Claims**

1. Disinfection and decontamination handwash, characterized in that it comprises an aqueous solution which contains

   a) 5 to 35 wt.% $C_1$-$C_{19}$ alkyl alcohol,
   b) 0.1 to 10.0 wt.% naturally occuring $C_6$-aryl-$C_1$-$C_8$ alkyl alcohol
   c) 0.5 to 45 wt.% sulphosuccinate, betaine or fatty alcohol ether sulphate or a mixture thereof, and
   d) 0.1 to 5.0 wt.% skin-compatible $\alpha$-hydroxy carboxylic acid as skin-protection component and for adjustment to a pH value of 2 to 7.

2. Disinfection and decontamination handwash according to claim 1, characterized in that it contains

   a) 10 to 30 wt.% $C_1$-$C_{19}$ alkyl alcohol,
   b) 0.5 to 5.0 wt.% naturally occurring $C_6$-aryl-$C_1$-$C_8$ alkyl alcohol,
   c) 0.5 to 15 wt.% sulphosuccinate, betaine or fatty alcohol ether sulphate or a mixture thereof, and
   d) 0.1 to 3.0 wt.% skin-compatible $\alpha$-hydroxy carboxylic acid.

3. Disinfection and decontamination handwash according to claim 1 or 2, characterized in that it contains

   a) as $C_1$-$C_{19}$ alkyl alcohol, ethanol, isopropanol or n-propanol or a mixture thereof,
   b) as naturally occurring $C_6$-aryl-$C_1$-$C_8$ alkyl alcohol, benzyl alcohol, phenyl ethyl alcohols, phenyl propyl alcohols or a mixture thereof,
   c) as sulphosuccinate, the disodium salt of sulphosuccinic acid lauryl ester with 1 to 4 EOs, as betaine, cocamidopropyl betaine, lauryl betaine or a mixture thereof; as fatty alcohol ether sulphate, sodium lauryl ether sulphate, magnesium lauryl ether sulphate, monoethanolamine ether sulphate or ammonium lauryl ether sulphate or a mixture thereof, each having 1 to 6 EOs and as
   d) $\alpha$-hydroxycarboxylic acid, lactic acid or mandelic acid.

4. Disinfection and decontamination handwash according to one of claims 1 to 3, characterized in that it contains

   | | |
   |---|---|
   | 15 wt.% | 1-propanol, |
   | 12 wt.% | 2-propanol, |
   | 2 wt.% | 2-phenylethyl alcohol, |
   | 10 wt.% | disodium salt of sulphosuccinic acid lauryl ester with 1 to 4 EOs, |
   | 3 wt.% | cocamidopropyl betaine, and |
   | 0.5 wt.% | lactic acid. |

5. Disinfection and decontamination handwash according to one of claims 1 to 3, characterized in that it contains

   | | |
   |---|---|
   | 10 wt.% | 1-propanol, |
   | 8 wt.% | 2-propanol, |
   | 2 wt.% | 3-phenylpropanol-1, |
   | 1 wt.% | disodium salt of sulphosuccinic acid lauryl ester with 1 to 4 EOs, |
   | 9 wt.% | sodium lauryl ether sulphate with 1 to 6 EOs, and |
   | 0.5 wt.% | lactic acid. |

6. Disinfection and decontamination handwash according to one of claims 1 to 3, characterized in that it contains

   | | |
   |---|---|
   | 10 wt.% | 1-propanol, |
   | 8 wt.% | 2-propanol, |

| | |
|---|---|
| 2 wt.% | 3-phenyl propanol-1 |
| 1 wt.% | disodium salt of sulphosuccinic acid lauryl ester with 1 to 4 EOs, |
| 9 wt.% | sodium lauryl ether sulphate with 1 to 6 EOs, and |
| 1 wt.% | mandelic acid. |

7. Disinfection and decontamination handwash according to one of claims 1 to 5, characterized in that the pH value lies in the range from 3 to

8. Process for the production of a disinfection and decontamination handwash according to one of claims 1 to 7, characterized in that the aryl alkyl alcohol is dissolved in the alkyl alcohol at room temperature and the other components are then added, the acid being added at the end.

9. Use of a solution according to one of claims 1 to 7 as a disinfection and decontamination handwash.

**Revendications**

1. Agent lavant de désinfection des mains et de décontamination des mains, caractérisé en ce qu'il comporte une solution aqueuse qui contient :

   a) 5 a 35 % en poids d'alcool alkylique $C_1$-$C_{19}$ ;
   b) 0,1 a 10,0 % en poids d'alcool aryl-$C_1$-$C_6$-alkylique-$C_1$-$C_8$ se trouvant dans la nature ;
   c) 0,5 a 45 % en poids de sulfosuccinate, de bétaïne ou d'éther sulfate d'alcool gras ou bien un mélange de ceux-ci ;
   d) 0,1 a 5,0 % en poids d'acide $\alpha$-hydroxycarboxylique compatible avec la peau, en tant que composant de protection de la peau et pour l'ajustement à une valeur de pH de 2 à 7.

2. Agent lavant de désinfection des mains et de décontamination des mains selon la revendication 1, caractérisé en ce qu'il contient :

   a) 10 a 30 % en poids d'alcool alkylique $C_1$-$C_{19}$ ;
   b) 0,5 a 5,0 % en poids d'alcool aryl-$C_6$-alkylique-$C_1$-$C_8$ se trouvant dans la nature ;
   c) 0,5 a 15 % en poids de sulfosuccinate, de bétaïne ou d'éther sulfate d'alcool gras ou bien un mélange de ceux-ci ; et
   d) 0,1 a 3,0 % en poids d'acide $\alpha$-hydrocarboxylique compatible avec la peau.

3. Agent lavant de désinfection des mains et de décontamination des mains selon la revendication 1 ou 2, caractérisé en ce qu'il contient en tant que :

   a) alcool - alkylique $C_1$-$C_{19}$, éthanol, isopropanol ou n-propanol ou bien un mélange de ceux-ci ;
   b) alcool aryl-$C_6$-alkylique-$C_1$-$C_8$ se présentant dans la nature, l'alcool benzylique, des alcools phényléthyliques, des alcools phénylpropyliques, ou bien un mélange de ceux-ci.
   c) Sulfosuccinate, le sel disodique de l'ester laurylique de l'acide sulfosuccinique avec 1 à 4 OE ;
   bétaïne, de la cocamidopropylbétaïne, de la laurylbétaïne ou bien un mélange de celles-ci ;
   éther sulfate d'alcool gras, du lauryléther sulfate de sodium, du lauryléther sulfate de magnésium, de l'éther sulfate de monoéthanolamine ou bien un mélange du lauryléther sulfate d'ammonium ou bien de ceux-ci à chaque fois avec 1 - 6 OE
   d) acide $\alpha$-hydroxycarboxylique, l'acide lactique ou l'acide mandélique.

4. Agent lavant pour la désinfection des mains et la décontamination de mains selon l'une des revendications 1 à 3, caractérisé en ce qu'il contient :

| | |
|---|---|
| 15 % | en poids de 1-propanol |
| 12 % | en poids de 2-propanol |
| 2 % | en poids d'alcool 2-phényléthylique |
| 10 % | en poids de sel disodique de l'ester laurylique de l'acide sulfosuccinique avec 1 à 4 OE |
| 3 % | en poids de cocamidopropylbétaïne et |
| 0,5 % | en poids d'acide lactique. |

5. Agent lavant pour la désinfection des mains et la décontamination de mains selon l'une des revendications 1 a 3, caractérisé en ce qu'il contient :

10 % en poids de 1-propanol
8 % en poids de 2-propanol
2 % en poids de 3-phénylpropanol-1
1 % en poids de sel disodique de l'ester laurylique de l'acide sulfosuccinique avec 1 à 4 OE
9 % en poids de lauryléther sulfate de sodium avec 1 - 6 OE
0,5 % en poids d'acide lactique

6. Agent lavant pour la désinfection des mains et la décontamination de mains selon l'une des revendications 1 à 3, caractérisé en ce qu'il contient :

10 % en poids de 1-propanol
8 % en poids de 2-propanol
2 % en poids de 3-phénylpropanol-1
1 % en poids de sel disodique de l'ester laurylique de l'acide sulfosuccinique avec 1 à 4 OE
9 % en poids de lauryléther sulfate de sodium avec 1 - 6 OE
1 % en poids d'acide mandélique.

7. Agent lavant de désinfection des mains et de décontamination des mains selon l'une des revendications 1 à 5, caractérisé en ce que la valeur du pH est dans la gamme de 3 à 6.

8. Procédé pour la production d'un agent lavant de désinfection des mains et de décontamination des mains selon l'une des revendications 1 à 7, caractérisé en ce que l'alcool arylalkylique est dissous à température ambiante dans l'alcool alkylique et ensuite les autres composants sont ajoutés, l'acide étant ajouté en dernier.

9. Utilisation d'une solution selon l'une des revendications 1 à 7 en tant qu'agent lavant de désinfection des mains et de décontamination des mains.